# EUROPEAN PATENT SPECIFICATION

(11) **EP 4 138 734 B1**
(45) Date of publication and mention of the grant of the patent: **05.03.2025**
(21) Application number: 21745498.2
(22) Date of filing: 22.06.2021
(51) Int. Cl.: A61F 2/24

(54) **PROSTHETIC IMPLANT SYSTEMS FOR DIAMETER ADAPTATION**
PROTHESENIMPLANTATSYSTEME ZUR DURCHMESSERANPASSUNG
SYSTÈMES D'IMPLANT PROTHÉTIQUE D'ADAPTATION DE DIAMÈTRE

(30) Priority: 23.06.2020 US 202063042734 P
(43) Date of publication of application: 01.03.2023
(73) Proprietor: Edwards Lifesciences Corporation, Irvine, CA 92614-5688 (US)
(72) Inventor: NIR, Noam, 30889 Caesarea (IL); BUKIN, Michael, 30889 Caesarea (IL)
(74) Representative: Eisenführ Speiser
(86) International application number: PCT/US2021/038420
(87) International publication number: WO 2021/262678

(56) References cited:
- US-A1- 2006 287 719
- US-A1- 2008 004 696
- US-A1- 2017 231 756

## Description

### RELATED APPLICATION

This application claims priority to, and the benefit of, U.S. Provisional Patent Application Serial No. 63/042,734, filed on June 23, 2020.

### BACKGROUND OF THE INVENTION

A variety of maladies may affect an individual's body. Such maladies may be of the individual's heart, and may include maladies of the individual's heart valves, including the aortic, mitral, tricuspid, and pulmonary valves. Stenosis, for example, is a common and serious valve disease that may affect the operation of the heart valves and an individual's overall well-being.

Implants may be provided that may replace or repair portions of a patient's heart. Prosthetic implants, such as prosthetic valves, may be provided to replace a portion of a patient's heart. Prosthetic aortic, mitral, tricuspid, and even pulmonary valves may be provided.

Implants may be deployed to the desired portion of the patient's body percutaneously, in a minimally invasive manner. Such deployment may occur transcatheter, in which a catheter may be deployed through the vasculature of an individual.

Such implants may have a range of working diameters that the implants are effectively utilized at. For example, a prosthetic heart valve may be able to effectively operate within a working diameter range. The range, however, may be relatively low, as care must be taken not to over-expand or under-expand a prosthetic heart valve. Over-expansion or under-expansion may lead to the inability of the valve leaflets of the prosthetic heart valve to coapt properly. Further issues may arise from an over-expansion or under-expansion of a prosthetic heart valve or other types of implants, including damage to the implant, dislodgement of the implant, or other issues arising from the implant being over-expanded or under-expanded.

To address such issues, practitioners typically select an implant from an array of implants having different working diameters or ranges of working diameters. The working diameter of the implant is typically selected according to the diameter of the treatment site within the patient's body. Due to variation in the possible diameter of the treatment site in the patient's body, however, a practitioner may keep a large number of different implants on hand, which may be expensive and cumbersome. Further, such implants may still be utilized within a range of working diameters upon implantation. For example, if the implant is to be implanted within a portion of the body having a diameter of 27 millimeters, the practitioner may have available a 30 millimeter implant and a 25 millimeter implant, and may select the 25 millimeter diameter implant to expand to a working diameter of 27 millimeters, because a 27 millimeter implant may be too expensive to separately stock.

US 2017/0231756 A1 relates to unexpandable docking stations for docking an expandable valve which can include a valve seat, one or more sealing portions, and one or more retaining portions. The valve seat can be unexpandable or substantially unexpandable beyond a deployed size. The one or more sealing portions are connected to the valve seat and extend radially outward of the valve seat. The one or more sealing portions are constructed to expand outward of the valve seat and provide a seal over a range of sizes. The one or more retaining portions are connected to the one or more sealing portions. The one or more retaining portions are configured to retain the docking station at a deployed position.

US 2006/0287719 A1 relates to a two-stage or component-based valve prosthesis. The prosthetic valve comprises a support structure that is deployed at a treatment site. The prosthetic valve further comprises a valve member configured to be quickly connected to the support structure. The support structure may take the form of a stent that is expanded at the site of a native valve. The support structure is provided with a coupling means for attachment to the valve member, thereby fixing the position of the valve member in the body.

US 2008/0004696 A1 relates to a cardiovascular valve assembly comprised of a base member and an exchangeable valve member detachably mountable thereto. The base member includes a tubular body. The valve member includes a valve frame that supports a plurality of valve leaflets. The diameter of the tubular body can be increased to receive an exchangeable valve member having larger dimensions.

### SUMMARY

The aspect of the presently claimed invention is set out in the independent claim. Particular embodiments of this aspect is set out in the dependent claims. Any subject matter contained herein that does not fall within the scope of the appended claims is considered as being useful for understanding the invention.

Improvements accordingly may be desired in addressing implants and the diameters of implants to be deployed to portions of a patient's body. Embodiments as disclosed herein may provide for increased flexibility of providing prosthetic implants having various working diameters, and for reducing expense associated with keeping a stock of prosthetic implants at various working diameters.

Disclosed herein is a prosthetic valve having a docking frame that may have a relatively large range of working diameters. A valve frame is configured to dock with the docking frame. The valve frame may have a working diameter that is closely tailored to the diameter of the implantation site. Further, the valve frame may have a lesser working diameter range than the working diameter range of the docking frame. As disclosed herein, the valve frame may be optimized to operate at the diameter of the implantation site.

Further, the docking frame may comprise a sturdier and robust structure than the valve frame, able to withstand the force exerted radially outward from the prosthetic valve during and following a deployment that may anchor the prosthetic valve in position. The valve frame accordingly may involve less material than a full prosthetic valve, and thus may be less expensive and complicated to manufacture. As such, a user may be able to stock a larger number and variation in the size of valve frames than may have been previously stocked with full prosthetic valves, due to a relatively reduced cost of the individual valve frames versus the full prosthetic valves.

As disclosed herein, the valve frame docks to the docking frame in a manner that locks the diameter and axial length of the docking frame in position. The valve frame accordingly is utilized to resist radial compression of the docking frame by the patient's body, and may maintain a force applied radially outward from the docking frame in an expanded state.

As disclosed herein, a prosthetic valve system includes a docking frame configured to be implanted within a portion of a patient's body. A valve frame may have an outer surface and an inner surface and may be configured to dock to the docking frame to support the valve frame within the portion of the patient's body. A plurality of valve leaflets are coupled to the valve frame and configured to extend radially inward from the inner surface of the valve frame.

As disclosed herein, a method may include deploying a docking frame within a portion of a patient's body. The method may include deploying a valve frame such that the valve frame is docked to the docking frame and the docking frame supports the valve frame within the portion of the patient's body, a plurality of valve leaflets being coupled to the valve frame and extending radially inward from an inner surface of the valve frame.

### BRIEF DESCRIPTION OF THE DRAWINGS

These and other features, aspects, and advantages are described below with reference to the drawings, which are intended to illustrate, but not to limit, the disclosure. In the drawings, like reference characters denote corresponding features consistently throughout similar embodiments.
FIG. 1 is a perspective view of a docking frame coupled to a deployment and locking mechanism of a delivery apparatus according to an embodiment of the present disclosure.
FIG. 2 is cross sectional schematic view of a valve frame according to an embodiment of the present disclosure.
FIG. 3 is a cross sectional schematic view of a docking frame deployed to a portion of patient's body according to an embodiment of the present disclosure.
FIG. 4 is a cross sectional schematic view of a valve frame positioned within a portion of patient's body in an unexpanded state according to an embodiment of the present disclosure.
FIG. 5 is a cross sectional schematic view of a valve frame docked to a docking frame in an expanded state according to an embodiment of the present disclosure.
FIG. 6 is a perspective view of a valve frame according to an embodiment of the present disclosure.
FIG. 7 is a cross sectional schematic view of the valve frame shown in FIG. 6 docked to a docking frame according to an embodiment of the present disclosure.
FIG. 8 is a cross sectional schematic view of a valve frame docked to a docking frame in an expanded state according to an embodiment of the present disclosure.
FIG. 9 is a cross sectional schematic view of the valve frame shown in FIG. 8 docked to a docking frame in an unexpanded state according to an embodiment of the present disclosure.
FIG. 10 is a cross sectional schematic view of a connector of a valve frame engaging a connector of a docking frame.
FIG. 11 is a cross sectional schematic view of a valve frame docked to a docking frame in an expanded state according to an embodiment of the present disclosure.
FIG. 12 is a plan view of a connector of the valve frame and the connector of the docking frame shown in FIG. 11 engaging.
FIG. 13 is a cross sectional schematic view of a valve frame and a docking frame in an unexpanded state according to an embodiment of the present disclosure.
FIG. 14 is a cross sectional schematic view of the valve frame docked to the docking frame shown in FIG. 13 in an expanded state according to an embodiment of the present disclosure.
FIG. 15 is a cross sectional schematic view of connectors of a valve frame and docking frame within a cover according to an embodiment of the present disclosure.
FIG. 16 is a cross sectional schematic view of a valve frame and a docking frame in an unexpanded state according to an embodiment of the present disclosure.
FIG. 17 is a perspective schematic view of a valve frame and a docking frame according to an embodiment of the present disclosure.
FIG. 18 is a side view of a delivery apparatus according to an embodiment of the present disclosure.

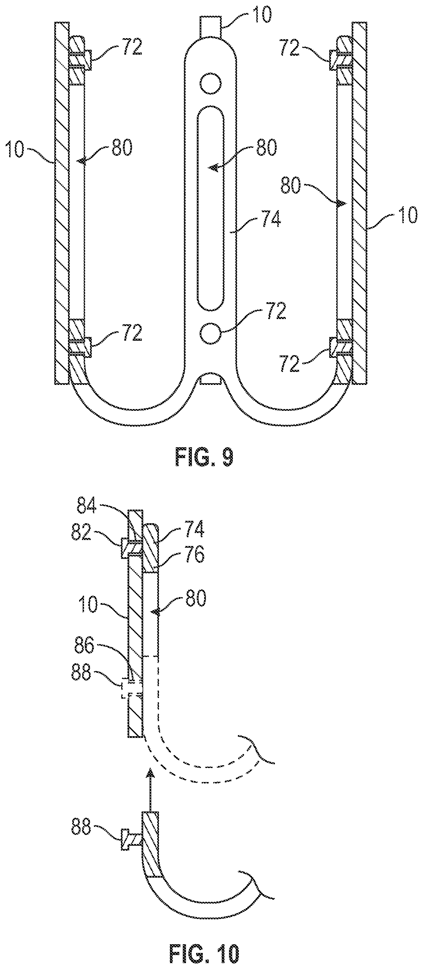

The invention relates to a prosthetic valve system as illustrated in fig. 10-16.

### DETAILED DESCRIPTION

The following description and examples illustrate some example embodiments of the disclosure in detail. Those of skill in the art will recognize that there are numerous variations and modifications of the disclosure that are encompassed by its scope. Accordingly, the description of a certain example embodiment should not be deemed to limit the scope of the present disclosure.

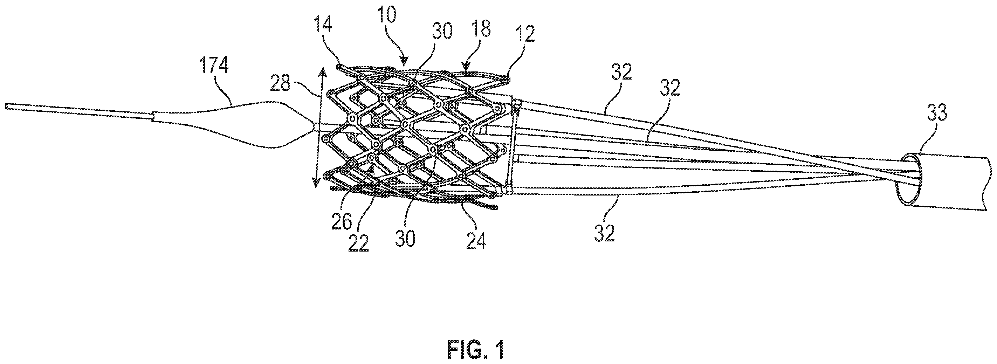

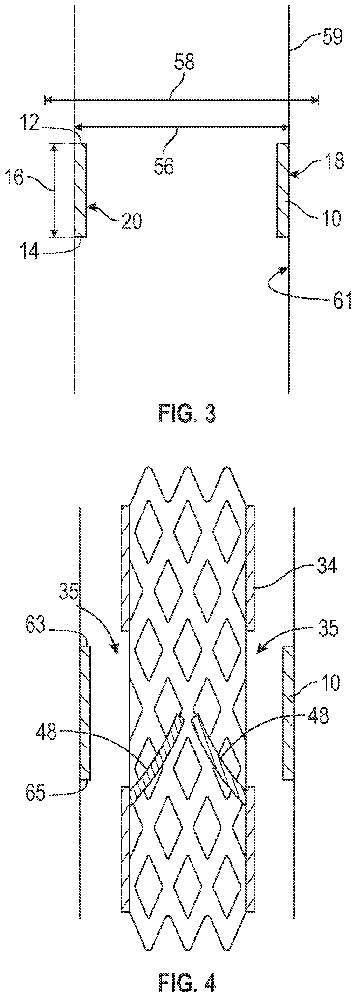

FIG. 1 illustrates a perspective view of an implant in the form of a docking frame 10 that may be utilized according to embodiments herein. The docking frame 10 may be utilized as part of a system that may comprise a prosthetic valve system. The docking frame 10 may include a proximal end 12 and a distal end 14 and may have a length 16 (marked in FIG. 3) therebetween. The docking frame 10 may be configured to be implanted within a portion of a patient's body.

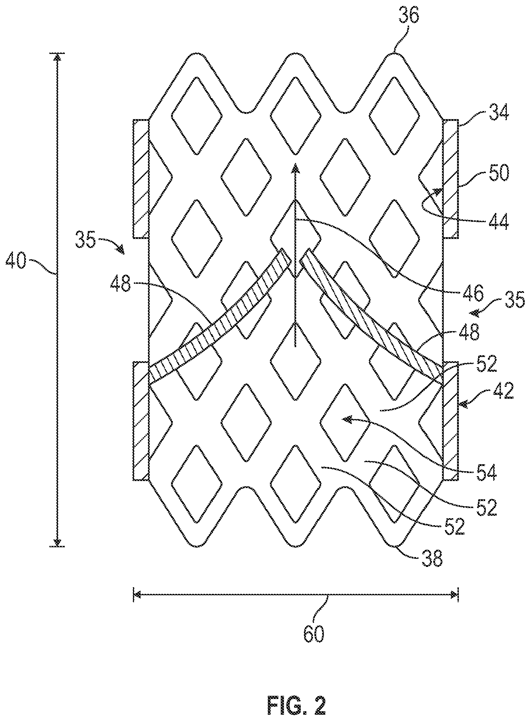

The docking frame 10 may have an outer surface 18 and an inner surface 20 (marked in FIG. 3). The outer surface 18 may be configured to contact a portion of a patient's body to which the docking frame 10 is implanted and may comprise an anchoring surface. The outer surface 18 may be configured to apply a radially outward force to the portion of the patient's body to which the docking frame 10 is implanted. The inner surface 20 may be configured to surround a valve frame that the docking frame 10 is docked to in embodiments, and may face an interior flow channel 46 of the valve frame (as marked in FIG. 2).

The docking frame 10 may include a frame body 22 that may be formed by a plurality of struts 24 separated by spaces in the forms of openings 26. The struts 24 may join together at junctures to form a repeating pattern of cells extending circumferentially about the axis that the frame body 22 surrounds.

The docking frame 10 may have a cylindrical shape as shown in FIG. 1 or may have a variety of other shapes as desired. For example, the docking frame 10 may have a "V" shape or bulb shape, or other shape, in embodiments as desired.

The docking frame 10 may be configured to be radially expandable. The docking frame 10 may be configured to move from a compressed (or unexpanded or undeployed) state to an expanded (or deployed) state. The docking frame 10 may be configured to expand radially outward from an axis that the frame body 22 surrounds. In embodiments, the outward radial expansion of the frame body 22 may result in the length 16 decreasing, with an increase in the width or diameter 28 of the docking frame 10. Such an operation may occur in a variety of manners.

For example, as shown in FIG. 1, the junctures of the struts 24 may comprise hinges 30. Each hinge 30 may allow the struts 24 to pivot about the hinge 30 such that a width of each of the openings 26 between the struts 24 increases as the length of the openings 26 decreases. Such a decrease in length and increase in width may allow the length 16 of the entire docking frame 10 to decrease and the width or diameter 28 to increase. The docking frame 10 may further be configured such that a reverse operation of decreasing the width of the openings 26 and increasing the length of the openings 26 allows the docking frame 10 to contract to the compressed, undeployed, or unexpanded state.

A variety of mechanisms may be utilized to control expansion and contraction of the docking frame 10. In embodiments, the docking frame 10 may comprise a mechanically expandable frame. As shown in FIG. 1, in embodiments a mechanism may be utilized to expand and contract the mechanically expandable frame, which may be a mechanical drive that couples to the docking frame 10. The mechanical drive may include a plurality of drive rods 32 that may couple to the docking frame 10 and be configured to rotate to control the expansion and contraction of the docking frame 10. Upon the docking frame 10 being expanded to the desired diameter, the drive rods 32 may decouple from the docking frame 10. Such an operation is disclosed in U.S. Patent No. 9,913,716, filed January 24, 2017 and issued March 13, 2018. FIGS. 1-20 of U.S. Patent No. 9,913,716, for example, illustrate such an operation and decoupling of a frame from drive rods. Further, such a mechanism may include a locking mechanism, as disclosed in U.S. Patent No. 9,913,716, which may be utilized to lock the position of the expansion of the docking frame 10.

The docking frame 10 may have a relatively large working diameter range. Such a large range may be allowed due to the construction of the docking frame 10, and due to the lack of valve leaflets coupled to the docking frame 10. As such, the docking frame 10 may be operably expanded to a relatively large range of working diameters and be effectively implanted within a patient's body. As an example, the working diameter range may be 14 millimeters in embodiments, or between working diameters of 32 millimeters and 18 millimeters. In other embodiments, greater or lesser working diameter ranges may be utilized, and the greatest working diameter (here 32 millimeters) and smallest working diameter (here 18 millimeters) may be greater or lesser in embodiments as desired. The greatest working diameter may be the greatest diameter that the docking frame 10 may operate effectively at, and the smallest working diameter may be the smallest diameter that the docking frame 10 may operate effectively at.

In embodiments, the docking frame 10 may be configured to be balloon expandable, or may be self-expanding, among other methods of expansion.

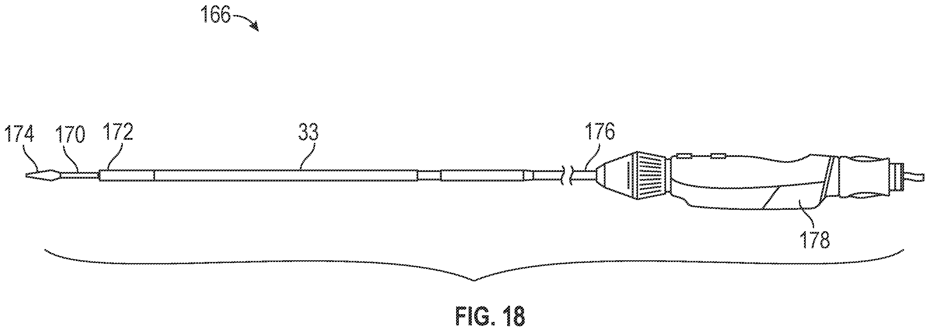

FIG. 1 illustrates the docking frame 10 coupled to a delivery shaft 33 of a delivery apparatus for the docking frame 10. A delivery apparatus that may be utilized is shown in FIG. 18, for example. Other forms of delivery apparatuses may be utilized as desired. The docking frame 10 may be released from the delivery shaft upon deployment, for example as disclosed in U.S. Patent No. 9,913,716 (as shown in FIGS. 1-20). The docking frame 10 may be positioned within the desired portion of the patient's body (e.g., within the patient's vasculature), and then expanded within such a portion. The delivery shaft 33 may then be withdrawn from the patient's body. Other methods of delivery may be utilized as desired.

FIG. 2 illustrates a cross sectional view of an embodiment of a valve frame 34 that may be utilized according to embodiments herein. The valve frame 34 may be utilized as part of a system that may comprise a prosthetic valve system. The valve frame 34 may be configured to dock to the docking frame 10 to support the valve frame 34 within the portion of the patient's body.

The valve frame 34 may include a proximal end 36, a distal end 38, and a length 40 therebetween. The valve frame 34 may have an outer surface 42 and an inner surface 44. The outer surface 42 may be configured to face outward, towards the surface of the implantation site of the patient's body, and may face towards the inner surface 20 of the docking frame 10 in embodiments. The inner surface 44 may face towards a flow channel 46 within the valve frame 34. In embodiments, the valve frame 34 may include openings 35 that the docking frame 10 may be positioned within. The openings 35 may be positioned in the body 50 of the valve frame 34 and may comprise cut-outs in the outer surface 42 of the valve frame 34.

A plurality of leaflets 48 may be coupled to the valve frame 34 and positioned within the flow channel 46. Two leaflets 48 are represented in FIG. 2, although in embodiments three leaflets or a greater number may be utilized as desired. The leaflets 48 may extend radially inward from the inner surface 44 of the valve frame 34. The leaflets 48 may be configured to move towards each other to close flow through the valve frame 34, and may be configured to move radially away from each other to open flow through the valve frame 34. The proximal end 36 of the valve frame 34 may comprise an outflow end, and the distal end 38 of the valve frame 34 may comprise an inflow end. The leaflets 48 may move between opened and closed states in a similar manner that native leaflets within a patient's body may perform. The leaflets 48 may control flow within the flow channel 46.

Similar to the docking frame 10, the valve frame 34 may be configured to be radially expandable. The valve frame 34 may include a frame body 50 that may be formed by a plurality of struts 52 separated by spaces in the forms of openings 54. The struts 52 may join together at junctures to form a repeating pattern of cells extending circumferentially about the axis that the valve frame 34 surrounds.

The valve frame 34 may be configured to move from a compressed (or unexpanded or undeployed) state to an expanded (or deployed) state. The valve frame 34 may be configured to expand radially outward from an axis that the frame body 50 surrounds. In embodiments, the radial expansion of the frame body 50 may cause the length 40 to decrease with an increase in the width or diameter 60 of the valve frame 34. Such an operation may occur in a variety of manners.

The junctures of the struts 52 may be flexible, or may comprise a hinge as disclosed in regard to FIG. 1. Each juncture may allow the struts 52 to pivot about the juncture such that a width of each of the openings 54 between the struts 52 increases as the length of the openings decreases. Such a decrease in length and increase in width may allow the length 40 of the entire valve frame 34 to decrease and the width or diameter 60 to increase. The valve frame 34 may further be configured such that a reverse operation of decreasing the width of the openings 54 and increasing the length of the openings 54 allows the valve frame 34 to contract to the undeployed or unexpanded state.

The valve frame 34 may have a cylindrical shape (half of which is shown in FIG. 2), similar to the docking frame 10 as shown in FIG. 1 or may have a variety of other shapes as desired. For example, the valve frame 34 may have a "V" shape or bulb shape in embodiments as desired.

The valve frame 34 may have a working diameter range. The range may extend between the greatest working diameter of the valve frame 34 and the smallest working diameter of the valve frame. In embodiments, the working diameter range of the docking frame 10 may be greater than a working diameter range of the valve frame 34. The working diameter range of the valve frame 34 may be less than the range of the docking frame 10 because leaflets 48 are coupled to the valve frame 34 that may be required to properly coapt during operation of the prosthetic valve, and thus the valve frame 34 may only operate within a defined range, which may be less than the range of operation of the docking frame 10. Further, the valve frame 34 may be less sturdy than the docking frame 10, which may reduce the amount that the valve frame 34 can expand and thus reduce working diameter range of the valve frame 34 relative to the working diameter range of the docking frame 10. For example, the valve frame 34 may have a working diameter range of three millimeters in embodiments (e.g., between 26 and 29 millimeters). In embodiments, the working diameter range may be less than three millimeters (e.g., two millimeter, one millimeter, or lesser), or may be greater. The greatest working diameter of the docking frame 10 may also be greater than the greatest working diameter of the valve frame 34, as the docking frame 10 may have an ability to operably expand to a greater diameter. The greatest working diameter of the valve frame 34 for example, may be 29 millimeters and the greatest working diameter of the docking frame 10 may be 32 millimeters for example. Other values may be utilized in embodiments as desired.

The valve frame 34 may be configured to be utilized with the docking frame 10 in a system within the patient's body. The docking frame 10 may be configured to be more robust and sturdy than the valve frame 34, and able to better withstand compressive forces applied to the system, which may be as a result of the radial expansion forces applied to the patient's body by the docking frame 10. The valve frame 34 may be configured to dock to the docking frame 10 and support the leaflets 48 within the portion of the patient's body.

The valve frame 34 may be configured to be docked to the docking frame 10 within the patient's body, or may be configured to be docked to the docking frame 10 external to the patient's body and prior to insertion of the docking frame 10 within the patient's body in embodiments.

FIGS. 3-5 illustrate features of a method of utilizing a system disclosed herein. The system may be deployed to a portion of a patient's body comprising the patient's vasculature, which may comprise the patient's arteries or other venous bodies. The system may be utilized as a prosthetic valve system, which may be utilized to replace or augment the operation of a native heart valve of the patient's body. The portion of the patient's body accordingly may comprise a native aortic valve, or in embodiments may comprise another valve such as the mitral, tricuspid, or pulmonary valve. The system may be deployed to the annulus of the native valve as desired, or another location.

FIG. 3 represents the system being deployed to the native aortic valve, although the structure of the native aortic valve is not shown for clarity. The walls of the aorta 59 are shown in FIG. 3, including a surface 61 of the aorta 59, or surface of the heart valve annulus, to which the system is deployed.

The system may be deployed in a sequence of steps, in which the docking frame 10 may be deployed within a portion of the patient's body first, with the valve frame 34 following. In other embodiments herein, the docking frame 10 may be deployed with the valve frame 34.

In embodiments, the system may be deployed in an implantation procedure without pre-visualization or pre-computed tomography (CT) being performed of the patient. In certain procedures, pre-CT is performed to determine the size of the patient's vasculature, to determine what size of implant to be implanted within the patient's vasculature. Such visualization is typically performed because the user (e.g., a medical clinician such as a surgeon) must select a size of implant to deploy within the patient's body and thus must know prior to the implantation procedure what is the size of the patient's vasculature. In embodiments herein however, the system may be deployed without such pre-visualization of the size of the patient's vasculature occurring. Such a feature may reduce the number of visits that the patient must make to a medical clinician (such as a medical imaging clinician), and may simplify the implantation process. Such a feature may be allowed as the docking frame 10 may be configured to expand to a variety of working diameters.

As such, referring to FIG. 3, the docking frame 10 may be passed into the patient's body mounted to a delivery apparatus (with a delivery shaft 33 of a delivery apparatus shown in FIG. 1). The docking frame 10 may then be deployed to the patient's vasculature and expanded radially outward to a diameter 56 as shown in FIG. 3. The outer surface 18 of the docking frame 10 may be radially pressed outward against the inner surface 61 of the patient's vasculature.

The docking frame 10 may be configured to expand to a variety of working diameters, and may be capable of expanding to a greater diameter if the surface of the patient's vasculature did not block the docking frame 10. Diameter 58 shown in FIG. 3, for example, represents a greatest working diameter of the docking frame 10 that the frame 10 may operably expand to. The docking frame 10, however, as shown in FIG. 3 only expands to the working diameter 56, as such a diameter fits the diameter of the patient's vasculature. As discussed, the docking frame 10 may further have a working diameter that is less than the diameter 56. During radial expansion of the docking frame 10, a medical clinician may visualize the deployment of the docking frame 10 via an imaging device (e.g., fluoroscopy, echocardiography, or a combination thereof) to determine if the docking frame 10 is deployed in the desired position of the patient's body.

The docking frame 10 may further be visualized during the implantation procedure to determine the diameter 56 that the docking frame 10 is expanded to. Such a diameter may be measured through the imaging devices, such that the medical clinician is aware of the diameter 56 of the docking frame 10. In embodiments, other methods to determine the diameter 56 may be utilized solely or in combination, including a force meter utilized by the delivery apparatus, or other method of determining a force applied by the docking frame 10 to the vasculature. In embodiments in which the docking frame 10 is mechanically deployed, a number of turns of the drive rods 32 may be measured and utilized to determine an amount of expansion of the docking frame 10, and the diameter 56 of the implantation site.

In embodiments, a locking mechanism may be utilized to hold the radial expansion of the docking frame 10, such as disclosed in U.S. Patent No. 9,913,716. In embodiments, the delivery apparatus that delivered the docking frame 10 may hold the docking frame 10 in position while the valve frame 34 is delivered to the docking frame 10. In embodiments, the docking frame 10 may be balloon expandable or self-expanding, and thus may hold in position within the patient's vasculature as the valve frame 34 is delivered.

A medical clinician may determine the diameter 56 that the docking frame 10 has expanded to, and thus may select a valve frame 34 to be deployed to the docking frame 10 based on the diameter 56. The valve frame 34 may be selected to have a working diameter that fits the determined diameter 56 that the docking frame 10 has expanded to (which may be the diameter of the implantation site). The selected working diameter of the valve frame 34 accordingly may be less than the greatest working diameter 58 that the docking frame 10 is capable of expanding outward to, as the diameter 56 is less than such a diameter 58.

The valve frame 34 selected may be tailored to function at the selected diameter 56. As such, the leaflets 48 of the valve frame 34 may be configured to function at the selected diameter 56, with effective coaptation between the leaflets 48. The working diameter of the valve frame 34 may fit the diameter 56. The possibility of over-expansion or under-expansion of the valve frame 34 is thus reduced. The valve frame 34 may be selected based on a measurement (e.g., via an imaging device) of the diameter of the portion of the patient's body to which the frame 34 is to be deployed. The valve frame 34 may further be selected based on the diameter of expansion of the docking frame 10 within the portion of the patient's body, which may be known via a force meter or other mechanical means of determining the diameter of expansion, among other methods.

The valve frame 34 may be selected from a set of a plurality of valve frames, which each may be configured to have a different greatest working diameter than each other. For example, a first valve frame in the set may have a greatest working diameter of 26 millimeters, a second valve frame in the set may have a greatest working diameter of 29 millimeters, and a third valve frame in the set may have a greatest working diameter of 32 millimeters. Each valve frame in the set may have a working diameter range of three millimeters for example (e.g., 23 millimeters to 26 millimeters for the first valve frame, 26 millimeters to 29 millimeters for the second valve frame, and 29 millimeters to 32 millimeters for the third valve frame). If the diameter 56 is 28 millimeters, then second valve frame in the set may accordingly be selected. The greatest working diameters and working diameter ranges may vary according to embodiments herein (e.g., may be greater or lesser as desired), and the working diameter ranges may overlap in embodiments as desired.

The user accordingly may have a set of a plurality of valve frames that may have working diameter ranges less than a certain amount (e.g., three millimeters), and may have working diameter ranges that are narrowly tailored to a specific working diameter. The user (e.g., a medical clinician) may then be able to select a valve frame with a working diameter that fits the diameter 56, and may be optimized to operate at that diameter 56. In certain embodiments, the user may select a valve frame with a narrow working diameter range (e.g., 1 millimeter or less) that may exactly fit and be optimized for the diameter 56. A large number of different valve frames may be included in the set of valve frames, however, it is possible that the expense of each valve frame may be less than the expense of an entire prosthetic valve, thus reducing costs associated with storing a large number of different valve frames relative to the cost of storing a large number of full prosthetic valves. As such, the diameter 56 may be adapted to, with the selection of the desired valve frame.

Further, the valve frames in the set may be less robust and sturdy than the docking frame 10, and thus may be less expensive to maintain a large store of different sized valve frames. The medical clinician accordingly may keep a variety of different sized valve frames at potentially a reduced expense than keeping a variety of different sized full prosthetic valves for deployment.

The valve frames in the set may each be configured to dock with the docking frame 10 to support the respective valve frame within the portion of the patient's body. Each valve frame may further include leaflets configured to operate at the working diameter range of the respective valve frame, and may extend radially inward from an inner surface of the respective valve frame. The docking frame 10 as such may be configured to dock with multiple different sizes of valve frames. The relatively large working diameter range of the docking frame 10 may allow the docking frame 10 to be deployed to a wide variety of diameters within the patient's body, with the valve frame being more closely tailored to the particular diameter within the patient's body.

Referring to FIG. 4, the selected valve frame 34 may be inserted in a compressed or undeployed configuration into the patient's vasculature. The valve frame 34 may be aligned with the docking frame 10 to dock with the docking frame 10. The openings 35, for example, of the valve frame 34 may be aligned with the docking frame 10 and may couple the valve frame 34 to the docking frame 10. The docking frame 10 may have a length in a deployed or expanded state that is less than the length of the valve frame 34 and may be sized to fit into the openings 35. As such, the valve frame 34 upon radial expansion may contact the upper edge 63 and lower edge 65 of the docking frame 10 to axially abut the docking frame 10 and dock with the docking frame 10. The length of the valve frame 34 may reduce to contact against the edges 63, 65 of the docking frame 10.

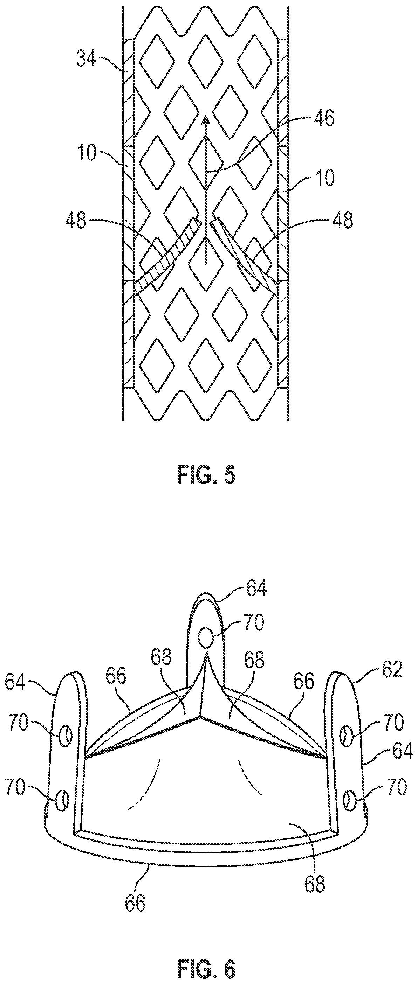

Such a docking is shown, for example, in FIG. 5. Such a docking may maintain the valve frame 34 in position and configured to resist axial forces of fluid flow applied to the valve frame 34. In other embodiments, other methods of docking may be utilized, such as pins or another form of connector that engages between the docking frame 10 and the valve frame 34.

FIG. 5 illustrates the valve frame 34 radially expanded and deployed and docked to the docking frame 10 and implanted within the patient's vasculature. The docking frame 10 supports the valve frame within the portion of the patient's body, and the valve frame 34 supports the leaflets 48 within the patient's body. The length of the valve frame 34 has reduced along the axis of the valve frame 34, and the diameter of the valve frame 34 has expanded to the working diameter 56. The valve leaflets 48 may operate within the patient's vasculature and may be utilized in lieu of native leaflets or other leaflets as desired.

The delivery apparatus utilized to deploy the docking frame 10 and the valve frame 34 may be removed from the patient's body, with the prosthetic valve system remaining implanted in place.

Variations in the systems disclosed herein may be provided.

FIG. 6, for example, illustrates an embodiment of a valve frame 62 including a plurality of axially extending support arms 64, which may be coupled to a plurality of leaflets 68 (three valve leaflets 68 are shown in FIG. 6). Each support arm 64 may couple to the valve leaflets 68 at a commissure of the leaflets 68, or in another position as desired. Each support arm 64 may extend from a proximal end of the valve frame 62 to a distal end of the valve frame 62. Three support arms 64 may be provided, circumferentially spaced from each other at the commissures. The support arms 64 may be equally spaced from each other circumferentially.

The valve frame 62 may include one or more circumferentially extending support arms 66 that may couple the axially extending support arms 64 to each other. The circumferentially extending support arms 66 may extend around the valve leaflets 68 at a base or distal portion of the valve leaflets 68, and may extend around the flow channel of the valve frame 62. The circumferentially extending support arms 66 may form an outer periphery of the valve frame 62. Openings may be otherwise positioned between the axially extending support arms 64, for instance the proximal ends of the arms 64.

The valve frame 62 may have an outer surface and an inner surface and configured to dock to the docking frame 10 to support the valve frame within a portion of the patient's body. Three leaflets 68 may couple to the valve frame 62 and extend radially inward from the inner surface of the support arms 64 as shown, or a greater or lesser number of leaflets 68 may be utilized as desired.

The valve frame 62 may be configured as a wire-form, which may be laser-cut, 3D printed, or formed in another manner. The valve frame 62 as such may be a less robust structure than the cylindrical valve frame shown in FIG. 2, as material may not extend between the central and proximal portions of the axially extending support arms 64.

The valve frame 62 may be configured to be radially collapsed and radially expanded, and may be expanded radially outward from the axis that the valve frame 62 surrounds. The valve frame 62 may be configured such that a length of the valve frame 62 decreases as the width or diameter of the valve frame 62 increases, as disclosed herein.

The valve frame 62 may include one or more connectors 70 that may be positioned on the axially extending support arms 64 or another portion of the valve frame 62 such as the circumferentially extending support arms 66. The connectors 70 may comprise openings as shown in FIG. 6, or may have another configuration in another embodiment, such as pins, latches, or other connectors as desired. The connectors 70 may be utilized to dock the valve frame 62 to a docking frame 10 such as the docking frame shown in FIG. 1. The docking frame 10 may be configured to have connectors 72 or the like.

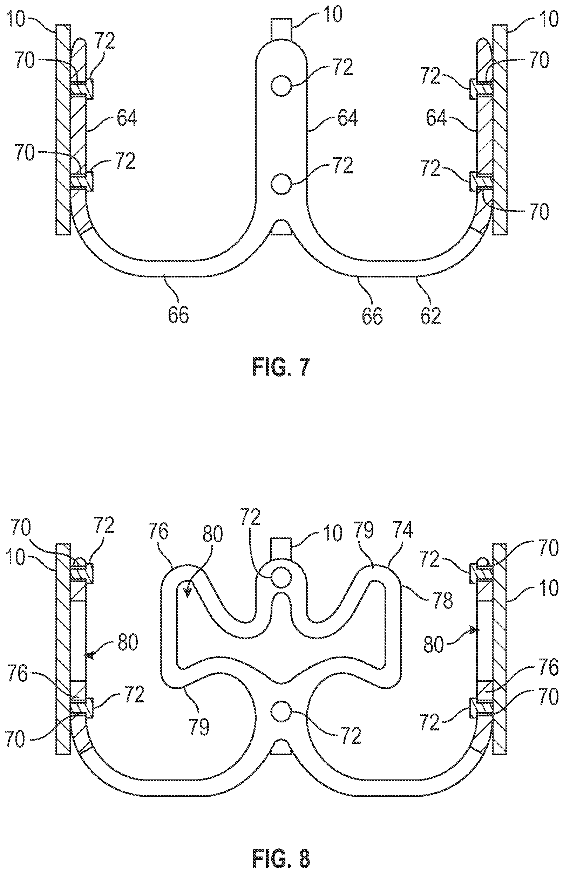

FIG. 7, for example, illustrates the docking frame 10 coupled to the valve frame 62 with connectors 72 in the form of pins extending through and engaging the connectors 70 of the valve frame 62. The docking frame 10 and valve frame 62 are shown in partial cross sectional view in FIG. 7. The leaflets 68 and a third support arm 66 are not shown for clarity. The connectors 72, 70 may include a proximal connector and a distal connector, and engagement of the connectors 72, 70 may be provided at each of the support arms 64.

The valve frame 62 and the docking frame 10 may be docked together by passing the connectors 70 of the docking frame 10 through the connectors 72 of the valve frame 62. The docking may occur prior to the valve frame 62 and docking frame 10 being inserted into the patient's body and external to the patient's body.

In such an embodiment, pre-CT or other forms of visualization of the implantation site may occur. A user such as a medical clinician may thus determine the diameter of the portion of the patient's body to which the system will be deployed. The user may then select a desired size of the valve frame 62 from a selection of possible sizes of valve frames, in a manner as disclosed herein. For example, the valve frame 62 selected may have a working diameter that fits the diameter of the portion of the patient's body to which the system will be deployed, and may be selected from a set of other valve frames having other working diameters, as disclosed herein. The user may then dock the selected valve frame 62 to the docking frame 10 via the connectors 72, 70. The docked valve frame 62 and docking frame 10 may then be inserted into the patient's body. The valve frame 62 may be deployed and radially expanded along with the docking frame 10, due to the coupling between the valve frame 62 and docking frame 10.

In embodiments, the valve frame may be utilized to resist radial compression of the valve frame and docking frame that may be applied to the frames by the portion of the patient's body to which the system is implanted. Such radial compression may result from the radial expansion of the frames, and the resistive force applied by the patient's body to such expansion. In embodiments, the valve frame may be configured to resist axial expansion of the docking frame to provide such a feature. FIG. 8, for example, illustrates an embodiment of a valve frame 74 configured to be a self-expanding valve frame and configured to expand radially outward. The valve frame 74 may be configured to apply a radial force outward due to the biasing of the valve frame. The valve frame 74 may be configured similarly as the valve frame 62 shown in FIG. 6 unless otherwise indicated.

The axially extending support arms 76, for example, may form a shaped body 78 that is configured to be expanded in length in a compressed or undeployed configuration, and be biased to be reduced in length and expanded in width in a deployed configuration. The shaped body 78 for example may surround an opening 80 in the support arms 76. The shaped body 78 may include wing shaped portions 79 that extend circumferentially. The axially extending support arms 76 may be coupled to each other with one or more circumferentially extending support arms, as shown in FIG. 6, for example. The valve frame 74 may be coupled to the docking frame 10 via the connectors 70, 72 on the valve frame 74 and docking frame 10 respectively.

Upon the frames being in a compressed state, the frames may be axially expanded and radially compressed. FIG. 9, for example, illustrates the valve frame 74 and docking frame 10 in a compressed configuration with a length being increased from the length shown in FIG. 8. The size of the opening 80 has expanded in the axial direction. Upon deployment of the frames, the docking frame 10 and valve frame 74 may move to the expanded configuration shown in FIG. 8. The valve frame 74 may resist axial expansion of the valve frame 74 and the docking frame 10 upon being docked to the docking frame 10. The shaped body 78, for instance, may provide an axial force that resists axial expansion of the valve frame 74 and docking frame 10. The frames may be resisted from expanding axially by the shaped body 78 and thus may be resisted from being radially compressed (as radial compression may require axial expansion). The valve frame 74 accordingly may serve to resist radial compression of the frames, and secure the frames within the patient's body.

The valve frame 74 may be selected in a similar manner as disclosed herein. For example, a user such as a medical clinician may select a desired size of the valve frame 74 from a selection of possible sizes of valve frames, in a manner disclosed herein. For example, the valve frame 74 selected may have a working diameter that fits the diameter of the portion of the patient's body to which the system will be deployed, and may be selected from a set of other valve frames having other working diameters, as disclosed herein.

In the embodiments of FIGS. 7-9, the valve frames 62, 74 may be docked to the docking frame 10 prior to insertion into the patient's body. The connectors 70, 72 for example, may be engaged between the docking frame 10 and the selected valve frame external to the patient's body.

In an embodiment such as shown in FIG. 10, the valve frame 74 may be coupled to the docking frame 10 prior to insertion into the patient's body with only a partial connection of connectors 86, 88.

FIG. 10 illustrates a side cross sectional view of a portion of the valve frame shown in FIGS. 8 and 9 comprising the support arm 76, and the docking frame 10 shown in FIGS. 8 and 9. In such a configuration, proximal connectors 82, 84 may couple to each other at a proximal end of the valve frame. Such proximal connectors 82, 84 may be engaged prior to insertion within the patient's body and external to the patient's body. Distal connectors 86, 88, however, may be unengaged prior to insertion. Upon expansion of the docking frame 10, the valve frame may then expand, causing the connector 88 to relatively move axially towards to the connector 86 to engage the connector 86. The engagement of the connectors 88, 86 may couple the docking frame 10 and valve frame to each other. The engagement may further serve as a locking mechanism that resists axial expansion of the docking frame 10 and the valve frame. The resistance of the axial expansion accordingly results in a resistance of radial compression of the docking frame 10 and the valve frame, as disclosed herein (as radial compression may require axial expansion).

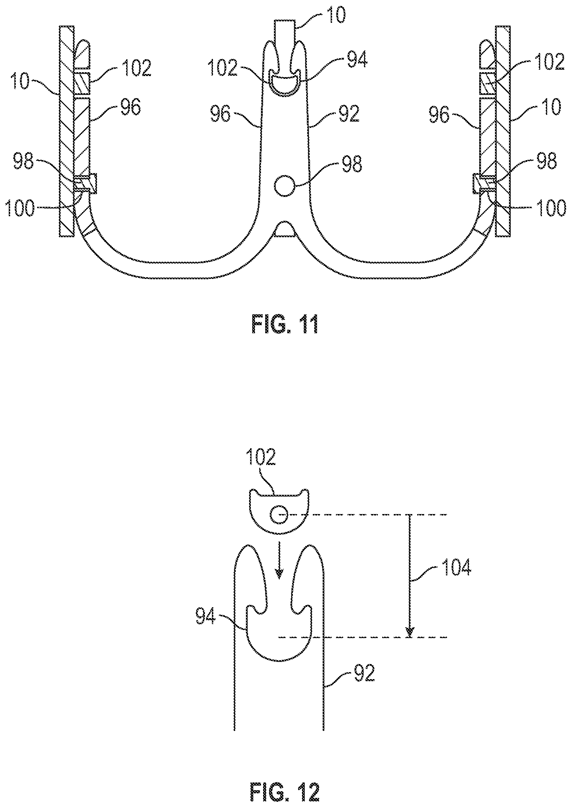

The system according the presently claimed invention utilize connectors that move axially relative to each other to couple the docking frame to the valve frame. Such connectors are utilized in a locking mechanism that may resist an axial expansion of the valve frame and docking frame, and accordingly result in a resistance to radial compression of the valve frame and docking frame. FIG. 11, for example, illustrates an embodiment of a valve frame 92 having a connector 94 in the form of an opening at a proximal portion of the valve frame axial support arm 96. The distal portion of the valve frame axial support arm 96 may include distal connectors 98, 100 engaged together prior to insertion into the patient's body and external to the patient's body. The valve frame 92 may be configured similarly as the valve frame 62 shown in FIG. 6 unless otherwise indicated.

A proximal connector 94 in the form of an opening may be configured to receive a proximal connector 102 in the form of a pin that is shaped to enter the opening in an axially distal direction and be impeded from exiting the opening in an axially proximal direction. The connector 94 is referred to as a first connector on the valve frame 92 and the connector 102 is referred to as a second connector on the docking frame 10. The first connector 94 is configured to relatively move axially towards the second connector 102 to engage the second connector 102 (and the second connector 102 is configured to relatively move axially towards the first connector 94 to engage the first connector 94).

FIG. 12, for example, illustrates the axial position of the connectors 94, 102 prior to engagement. The docking frame 10 and the valve frame 92 may both be in a compressed state in FIG. 12, prior to radial expansion. Due to radial expansion of the valve frame 92, the connector 94 moves axially towards to the connector 102 such that the connector 102 enters and is retained by the connector 94.

Notably, the axial distance 104 between the connectors 102, 94, or the length of axial distance travelled by the connector 102 relative to the connector 94, may define an amount that the length that the docking frame 10 and valve frame 92 may be shortened during compression, and accordingly defines a corresponding diameter of outward radial expansion of the valve frame 92 and docking frame 10 (as radial expansion may require axial shortening). The engagement of the connectors 102, 94 may resist further radial expansion of the valve frame 92 and the docking frame 10 beyond such a diameter.

Further, because the connector 102 cannot slide out of the connector 94, the axial distance 104 between the connector 102 and the connector 94 defines a diameter at which radial compression of the valve frame 92 and the docking frame 10 is resisted by the engagement. The engagement of the connectors 102, 94 resists axial expansion and thus resists radial compression of the valve frame and docking frame (as radial compression may require axial expansion). A radially inward force applied to the frames 92, 10 shown in FIG. 11 will not cause an axial expansion of the frames 92, 10 because the connector 102 is prevented from disengaging with the connector 94 axially.

In embodiments, the connector 94 may be moved axially proximal to engage the connector 102 with a tether of a delivery apparatus or the like. The tether may comprise a portion of the delivery apparatus utilized to deliver the docking frame 10 and valve frame 92 to the desired implantation site. The tether may be coupled to the valve frame 92. The tether may relatively move the connector 94 axially towards the connector 102 to engage the connectors 94, 102 within the patient's body.

During such an operation, the connectors 98, 100 may remain engaged with each other upon axial movement of the connectors 94, 102 relative to each other.

In embodiments, the axial distance 104 between the connectors 102, 94 may be selected based on a desired amount of radial expansion of the valve frame 92. If a greater amount of expansion may be desired, then the connector 94 may be positioned further distal on the valve frame 92, and if a lesser amount of expansion is desired, then the connector 94 may be positioned further proximal on the valve frame 92. A valve frame 92 may be selected from a set of valve frames, each having a different axial position of a connector 94, which may correspond to a desired working diameter of the valve frame 92. The selection of the valve frame may occur from the set of valve frame according to methods disclosed herein.

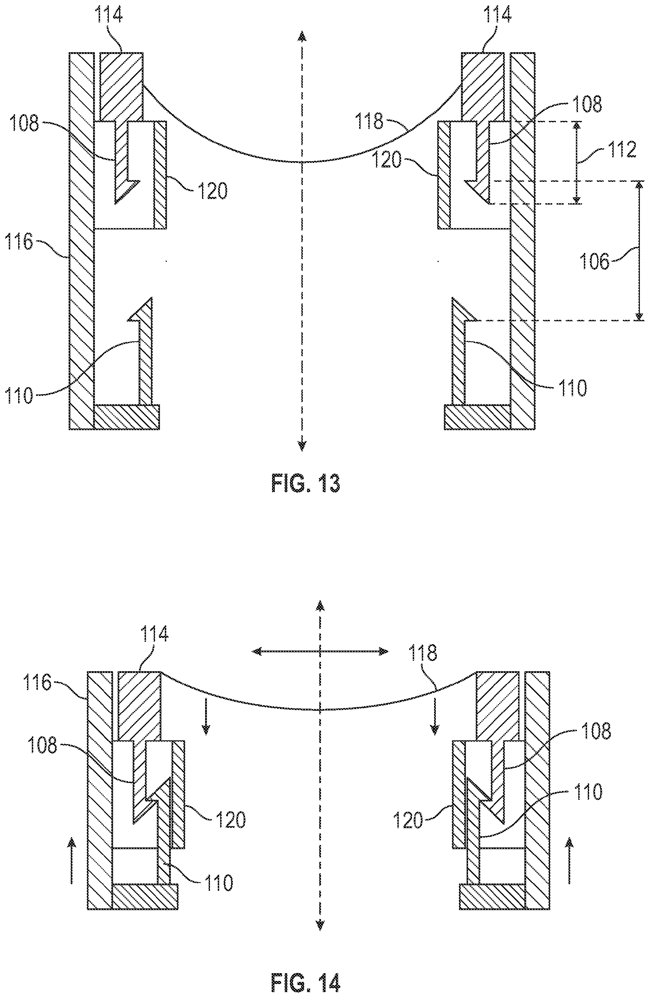

FIG. 13, for example, illustrates an embodiment in which an axial distance 106 or length of axial movement of connectors 108, 110 may be set based on a length 112 of the connector 108. A valve frame 114 is shown in FIG. 13 positioned upon a docking frame 116. A plurality of leaflets 118 may be coupled to the valve frame 114 (one leaflet 118 is represented in FIG. 13). The valve frame 114 may include connectors 108 in the form of arms having latches at their ends that extend axially distal. The arms may extend axially within covers in the form of sleeves 120 of the docking frame 116. Upon radial expansion (and axial shortening) of the docking frame 116 and valve frame 114, the connectors 108 may be relatively moved axially towards the connectors 110 on the docking frame 116 to engage the connectors 110.

FIG. 14, for example, illustrates the docking frame 116 and the valve frame 114 in a radially expanded state with the connectors 108, 110 moved axially towards each other and engaged with each other. The engagement may prevent the valve frame 114 and docking frame 116 from moving axially away from each other, thus preventing the length of the valve frame 114 and docking frame 116 from increasing. As such, the diameter of the valve frame 114 and docking frame 116 is resisted from decreasing, thus locking the valve frame 114 and docking frame 116 at the desired working diameter. The connectors 108, 110 accordingly comprise a locking mechanism that resists axial expansion of the frames 116, 114 and resists radial compression of the frames 116, 114.

In embodiments, the length 112 of the connector 108 may be set according to the selected diameter of expansion for the valve frame 114. As such, the length 112 of the arm of the connector 108 may define a diameter at which radial compression of the valve frame and docking frame may be resisted by the locking mechanism, and may be tailored based on the desired working diameter of expansion of the valve frame 114. Different valve frames having different diameters may accordingly be utilized with the same docking frame 116, with a resistance to radial compression being defined at a diameter set by the length 112 of the connector 108.

The valve frame 114 may be selected in a similar manner as disclosed herein. For example, a user (e.g., a medical clinician) may select a desired size of the valve frame 114 from a selection of possible sizes of valve frames, as disclosed herein. For example, the valve frame 114 selected may have a working diameter that fits the diameter of the portion of the patient's body to which the system will be deployed, and may be selected from a set of other valve frames having other working diameters, as disclosed herein. The length of the connector 108 may be configured to correspond to the desired working diameter for the valve frame 114. For example, the plurality of valve frames 114 may each have a connector 108 (such as an arm) that has a length that differs from each other and defines a diameter at which radial compression of the respective valve frame and the docking frame 116 is resisted.

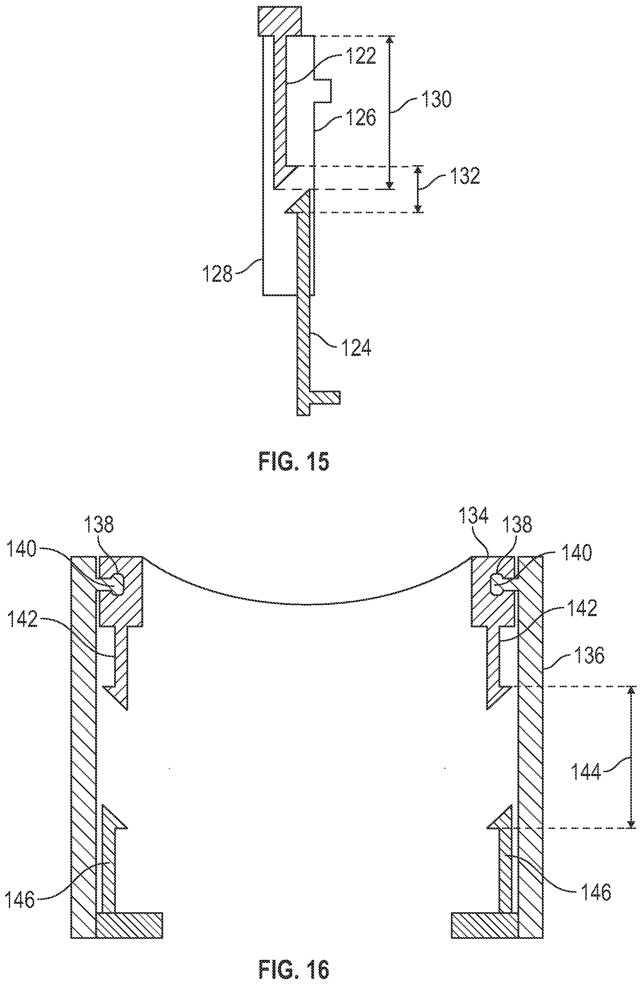

FIG. 15 illustrates an alternative embodiment in which the connectors 122, 124 on the valve frame and docking frame respectively may both be positioned in a cover 126 (such as a sleeve) of the valve frame 128. The cover 126 may impede the ability of the connectors 122, 124 to be snagged or snared or otherwise undesirably contact a portion of the patient's body or the valve system. The length 130 of the connector 122 may be set to correspond to a desired length 132 of axial movement of the connectors 122, 124, as discussed herein.

FIG. 16 illustrates an embodiment in which a proximal portion of the valve frame 134 engages a proximal portion of the docking frame 136 via a connector 138 on the valve frame 134 in the form of an opening that receives a connector 140 on the docking frame 136 in the form of a pin. The engagement of the connection may be performed exterior to the patient's body, before implantation. The engagement of the connectors 142, 146 may occur within the patient's body upon expansion of the frames, as disclosed herein. The length of the connector 142 of the valve frame 134 may be set to provide a desired axial distance 144 or length of axial movement of the connectors 142, 146, as discussed herein.

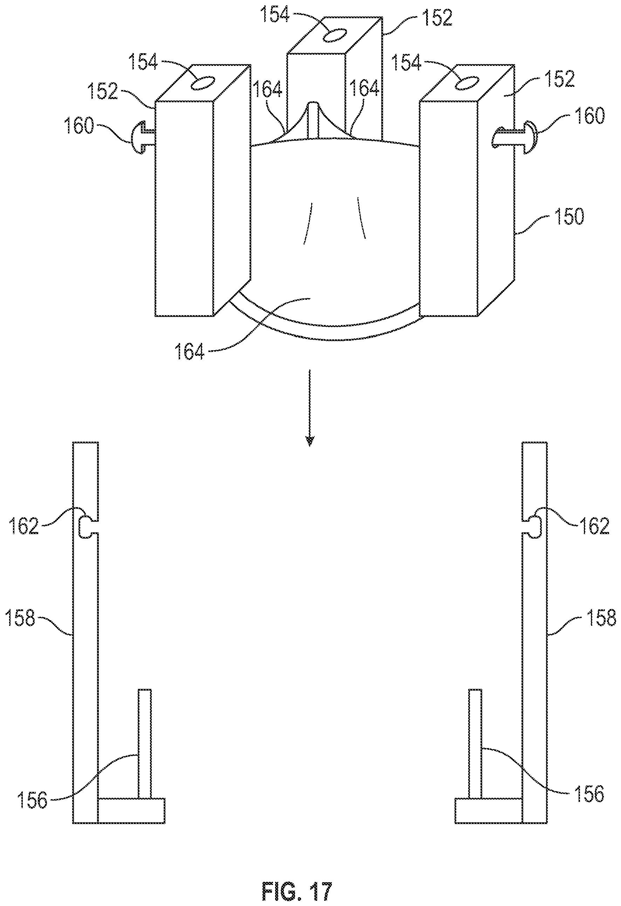

FIG. 17 illustrates an embodiment of a valve frame 150 in which the support arms 152 each include a connector 154 configured to axially slide upon a connector 156 in the form of a pin extending from a docking frame 158. The connectors 154 may comprise openings extending axially and configured to receive the pins. Upon sliding upon the pins, connectors 160 in the form of pins may engage connectors 162 in the form of openings in the docking frame 158. The valve frame 150 may be docked to the docking frame 158 by the connection of the connectors 154, 156, 160, 162 and the leaflets 164 may then operate upon deployment. The docking may occur exterior to the patient's body and prior to implantation as desired. In embodiments, the docking may occur within the patient's body.

Features of the docking frames and valve frames may be combined, substituted, and modified across embodiments.

FIG. 18 illustrates an embodiment of a delivery apparatus 166 that may be utilized according to embodiments herein. The delivery apparatus utilized may be varied in other embodiments. The delivery apparatus 166 may include the delivery shaft 33, which may include an implant retention area 170 that may retain one or more of the valve frame or docking frame. A sheath of the delivery shaft 33 may cover the docking frame or valve frame in the implant retention area 170. The implant retention area 170 may be positioned at a distal end 172 of the delivery shaft 33. A nosecone 174 (as marked in FIG. 1) may further be positioned at the distal end 172 of the delivery shaft 33. The delivery shaft 33 may include a proximal end 176 that may couple to a handle 178 utilized to grip the delivery apparatus 166, or may couple to another form of housing.

The embodiments as disclosed herein may be discussed in regard to a prosthetic valve, however, the systems, devices, and methods disclosed herein are not limited to prosthetic valves. Other forms of implants and prosthetic implants may utilize the systems, devices, and methods disclosed herein, including stents and other forms of medical implants.

The systems, devices, and methods disclosed herein are not limited to treatment of the aortic valve, but may extend to mitral, pulmonary, and tricuspid valves, as well as treatment of other portions of a patient's body. Other uses may be provided.

The implants may be cylindrical implants, or in other embodiments may have other shapes such as "V" shaped implants or other shapes as desired. The implants may be configured to expand radially outward from an axis that the implant surrounds, for example a longitudinal axis of the implant. The implants may be balloon expandable, mechanically expandable, or may be self-expanding in embodiments, unless otherwise indicated. The delivery apparatuses utilized, for example, may be configured to produce the desired form of expansion. For a balloon expandable valve, for example, the delivery apparatus may include an expansion balloon and may include a lumen for inflating and expanding the balloon positioned interior of the valve. For a mechanically expandable valve, the delivery apparatus may include a mechanical deployment mechanism for expanding the valve. For a self-expanding valve, the delivery apparatus may include a retractable sheath or the like for uncovering the valve and allowing the valve to expand. Other forms of deployment and delivery apparatuses may be utilized as desired.

The connectors as disclosed herein may comprise one or more of a pin, a latch, or an opening, among other forms of connectors.

The systems, devices, and methods disclosed herein may be used in a variety of procedures, which may include transcatheter aortic valve implantation (TAVI). The delivery apparatus and the systems disclosed herein may be utilized for transarterial access, including transfemoral access, to a patient's heart. The approach to the delivery site may be in a variety of manners. For example, an approach to a native aortic valve may be through an aortic arch. In embodiments, a ventricular approach may be utilized, approaching the native aortic valve from the inflow side of the native aortic valve.

In embodiments, the systems, devices, and method disclosed herein may be utilized for mitral, tricuspid, and pulmonary replacement and repair as well. The delivery systems may be utilized in transcatheter percutaneous procedures, including transarterial procedures, which may be transfemoral or transjugular. Transapical procedures, among others, may also be utilized.

The methods herein are not limited to the methods specifically described, and may include methods of utilizing the systems and devices disclosed herein.

The features of the embodiments disclosed herein may be implemented independently of other components disclosed herein. The various apparatuses of the systems may be implemented independently.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

Certain embodiments of systems, apparatuses, and methods are described herein, including the best mode known to the inventors for carrying out the same. Of course, variations on these described embodiments will become apparent to those of ordinary skill in the art upon reading the foregoing description. The inventor expects skilled artisans to employ such variations as appropriate, and the inventors intend for the systems, apparatuses, and methods to be practiced otherwise than specifically described herein..

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses an approximation that may vary, yet is capable of performing the desired operation or process discussed herein.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed. No language in the present specification should be construed as indicating any non-claimed element essential to the practice of the systems, apparatuses, and methods.

All patents, patent publications, and other publications referenced and identified in the present specification are for the purpose of describing and disclosing, for example, the compositions and methodologies described in such publications that might be used in connection with the systems, apparatuses, and methods. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

## Claims

1. A prosthetic valve system comprising:
a docking frame (10) configured to be implanted within a portion of a patient's body;
a valve frame (34) having an outer surface (42) and an inner surface (44) and configured to dock to the docking frame to support the valve frame within the portion of the patient's body;
a plurality of valve leaflets (48) coupled to the valve frame and configured to extend radially inward from the inner surface of the valve frame; and
a locking mechanism including a first connector (94) on the valve frame and a second connector (102) on the docking frame configured to relatively move axially towards the first connector to engage the first connector;
wherein an axial distance (104) between the first connector and the second connector defines a diameter (56) at which radial compression of the valve frame and of the docking frame is resisted by the locking mechanism; and
further comprising a third connector (100) on the valve frame and a fourth connector (98) on the docking frame, the third connector and fourth connector configured to remain engaged with each other upon axial movement of the second connector relative to the first connector.

2. The prosthetic valve system of claim 1, wherein the docking frame is a mechanically expandable frame.

3. The prosthetic valve system of claim 1 or claim 2, wherein the docking frame is radially expandable and the valve frame is radially expandable, and a working diameter range of the docking frame is greater than a working diameter range of the valve frame.

4. The prosthetic valve system of any of claims 1-3, wherein the docking frame is radially expandable and the valve frame is radially expandable, and a greatest working diameter (58) of the docking frame is greater than a greatest working diameter of the valve frame.

5. The prosthetic valve system of any of claims 1-4, further comprising a plurality of valve frames, each of the plurality of valve frames having a different greatest working diameter than each other and being configured to dock to the docking frame to support the respective valve frame within the portion of the patient's body, a plurality of valve leaflets (68) being coupled to the respective valve frame and configured to extend radially inward from an inner surface (44) of the respective valve frame.

6. The prosthetic valve system of any of claims 1-5, wherein the valve frame is configured to be docked to the docking frame within the patient's body.

7. The prosthetic valve system of any of claims 1-6, wherein the valve frame includes a plurality of axially extending support arms (64) coupled to the plurality of valve leaflets,
optionally further comprising one or more circumferentially extending support arms (66) coupling the plurality of axially extending support arms to each other.

8. The prosthetic valve system of any of claims 1-7, wherein the valve frame is self-expanding and configured to expand radially outward.

9. The prosthetic valve system of any preceding claim, wherein the second connector (102) is configured to relatively move axially towards the first connector (94) to engage the first connector due to radial expansion of the valve frame.

10. The prosthetic valve system of any preceding claim, wherein:
a.) the locking mechanism is configured to resist axial expansion of the docking frame; and/or
b.) the locking mechanism is configured to resist radial compression of the valve frame and of the docking frame; and/or
c.) the locking mechanism resists radial expansion of the valve frame beyond a diameter (56).

11. The prosthetic valve system of any preceding claim, wherein the first connector includes an arm extending axially (108), a length (112) of the arm defining a diameter at which radial compression of the valve frame and of the docking frame is resisted by the locking mechanism.

12. The prosthetic valve system of any preceding claim, further comprising a plurality of valve frames, each of the plurality of valve frames including a connector having an arm (108) configured to relatively move axially towards the second connector (110) to engage the second connector, each respective arm having a length (112) that differs from each other and that defines a diameter at which radial compression of the respective valve frame and of the docking frame is resisted.

13. The prosthetic valve system of any preceding claim, wherein the first connector or the second connector comprise one or more of a pin (160), a latch, or an opening (162).

## Patentansprüche

1. Klappenprothesensystem, umfassend:
einen Andockrahmen (10), der ausgestaltet ist, um innerhalb eines Abschnitts eines Körpers eines Patienten implantiert zu werden;
einen Klappenrahmen (34) mit einer Außenoberfläche (42) und einer Innenoberfläche (44), und der ausgestaltet ist, um an den Andockrahmen anzudocken, um den Klappenrahmen innerhalb des Abschnitts des Körpers des Patienten zu halten;
eine Vielzahl von Klappensegeln (48), die an den Klappenrahmen gekoppelt ist und ausgestaltet ist, um sich von der Innenoberfläche des Klappenrahmens radial einwärts zu erstrecken; und
einen Arretiermechanismus, der einen ersten Konnektor (94) auf dem Klappenrahmen und einen zweiten Konnektor (102) auf dem Andockrahmen einschließt, der ausgestaltet ist, um sich relativ axial in Richtung des ersten Konnektors zu bewegen, um in Eingriff mit dem ersten Konnektor zu kommen;
wobei ein axialer Abstand (104) zwischen dem ersten Konnektor und dem zweiten Konnektor einen Durchmesser (56) definiert, bei dem durch den Arretiermechanismus radialer Kompression des Klappenrahmens und des Andockrahmens Widerstand entgegengesetzt wird; und
des Weiteren umfassend einen dritten Konnektor (100) auf dem Klappenrahmen und einen vierten Konnektor (98) auf dem Andockrahmen, wobei der dritte Konnektor und der vierte Konnektor ausgestaltet sind, um bei axialer Bewegung des zweiten Konnektors relativ zu dem ersten Konnektor in Eingriff miteinander zu bleiben.

2. Klappenprothesensystem nach Anspruch 1, wobei der Andockrahmen ein mechanisch expandierbarer Rahmen ist.

3. Klappenprothesensystem nach Anspruch 1 oder Anspruch 2, wobei der Andockrahmen radial expandierbar ist und der Klappenrahmen radial expandierbar ist und ein Arbeitsdurchmesserbereich des Andockrahmens größer als ein Arbeitsdurchmesserbereich des Klappenrahmens ist.

4. Klappenprothesensystem nach einem der Ansprüche 1 bis 3, wobei der Andockrahmen radial expandierbar ist und der Klappenrahmen radial expandierbar ist, und ein größter Arbeitsdurchmesser (58) des Andockrahmens größer als ein größter Arbeitsdurchmesser des Klappenrahmens ist.

5. Klappenprothesensystem nach einem der Ansprüche 1 bis 4, des Weiteren umfassend eine Vielzahl von Klappenrahmen, wobei jeder von der Vielzahl der Klappenrahmen einen anderen größten Arbeitsdurchmesser als jeder andere aufweist und ausgestaltet ist, um an den Andockrahmen anzudocken, um den jeweiligen Klappenrahmen innerhalb des Abschnitts des Körpers des Patienten zu halten, wobei eine Vielzahl von Klappensegeln (68) an den jeweiligen Klappenrahmen gekoppelt ist und ausgestaltet ist, um sich von einer Innenoberfläche (44) des jeweiligen Klappenrahmens radial einwärts zu erstrecken.

6. Klappenprothesensystem nach einem der Ansprüche 1 bis 5, wobei der Klappenrahmen ausgestaltet ist, um innerhalb des Körpers des Patienten an den Andockrahmen angedockt zu werden.

7. Klappenprothesensystem nach einem der Ansprüche 1 bis 6, wobei der Klappenrahmen eine Vielzahl sich axial erstreckender Haltearme (64) einschließt, die an die Vielzahl von Klappensegeln gekoppelt ist,
gegebenenfalls des Weiteren umfassend einen oder mehrere sich im Umfang erstreckende Haltearme (66), die die Vielzahl der sich axial erstreckenden Haltearme aneinander koppelt bzw. koppeln.

8. Klappenprothesensystem nach einem der Ansprüche 1 bis 7, wobei der Klappenrahmen selbstexpandierend ist und ausgestaltet ist, um radial auswärts zu expandieren.

9. Klappenprothesensystem nach einem der vorhergehenden Ansprüche, wobei der zweite Konnektor (102) ausgestaltet ist, um sich relativ axial in Richtung des ersten Konnektors (94) zu bewegen, um aufgrund von radialer Expansion des Klappenrahmens in Eingriff mit dem ersten Konnektor zu kommen.

10. Klappenprothesensystem nach einem der vorhergehenden Ansprüche, wobei:
a.) der Arretiermechanismus ausgestaltet ist, um axialer Expansion des Andockrahmens Widerstand entgegenzusetzen; und/oder
b.) der Arretiermechanismus ausgestaltet ist, um radialer Kompression des Klappenrahmens und des Andockrahmens Widerstand entgegenzusetzen; und/oder
c.) der Arretiermechanismus radialer Expansion des Klappenrahmens über einen Durchmesser (56) hinaus Widerstand entgegensetzt.

11. Klappenprothesensystem nach einem der vorhergehenden Ansprüche, wobei der erste Konnektor einen Arm einschließt, der sich axial (108) erstreckt, wobei eine Länge (112) des Arms einen Durchmesser definiert, bei dem der Arretiermechanismus radialer Kompression des Klappenrahmens und des Andockrahmens Widerstand entgegensetzt.

12. Klappenprothesensystem nach einem der vorhergehenden Ansprüche, des Weiteren umfassend eine Vielzahl von Klappenrahmen, wobei jeder von der Vielzahl der Klappenrahmen einen Konnektor einschließt, der einen Arm (108) aufweist, der ausgestaltet ist, um sich relativ axial in Richtung des zweiten Konnektors (110) zu bewegen, um in Eingriff mit dem zweiten Konnektor zu kommen, wobei jeder jeweilige Arm eine Länge (112) aufweist, die sich von derjenigen der jeweiligen anderen unterscheidet, und die einen Durchmesser definiert, bei der radialer Kompression des jeweiligen Klappenrahmens und des Andockrahmens Widerstand entgegengesetzt wird.

13. Klappenprothesensystem nach einem der vorhergehenden Ansprüche, wobei der erste Konnektor oder der zweite Konnektor ein oder mehrere von einem Stift (160), einer Verriegelung oder einer Öffnung (162) umfassen.

## Revendications

1. Système de valvule prothétique comprenant :
un cadre d'ancrage (10) conçu pour être implanté à l'intérieur d'une partie du corps d'un patient ;
un cadre (34) de valvule présentant une surface externe (42) et une surface interne (44) et conçu pour s'ancrer au cadre d'ancrage afin de supporter le cadre de valvule à l'intérieur de la partie du corps du patient ;
une pluralité de feuillets valvulaires (48) accouplés au cadre de valvule et conçus pour s'étendre radialement vers l'intérieur depuis la surface interne du cadre de valvule ; et
un mécanisme de verrouillage comprenant un premier élément de liaison (94) sur le cadre de valvule et un deuxième élément de liaison (102) sur le cadre d'ancrage conçu pour se déplacer relativement axialement vers le premier élément de liaison afin de venir en prise avec le premier élément de liaison ;
une distance axiale (104) entre le premier élément de liaison et le deuxième élément de liaison définissant un diamètre (56) au niveau duquel une compression radiale du cadre de valvule et du cadre d'ancrage est contrée par le mécanisme de verrouillage ; et
comprenant en outre un troisième élément de liaison (100) sur le cadre de valvule et un quatrième élément de liaison (98) sur le cadre d'ancrage, le troisième élément de liaison et le quatrième élément de liaison étant conçus pour rester en prise l'un avec l'autre lors d'un mouvement axial du deuxième élément de liaison par rapport au premier élément de liaison.

2. Système de valvule prothétique selon la revendication 1, le cadre d'ancrage étant un cadre mécaniquement déployable.

3. Système de valvule prothétique selon la revendication 1 ou la revendication 2, le cadre d'ancrage étant radialement déployable et le cadre de valvule étant radialement déployable et une plage de diamètre de travail du cadre d'ancrage étant supérieure à une plage de diamètre de travail du cadre de valvule.

4. Système de valvule prothétique selon l'une quelconque des revendications 1 à 3, le cadre d'ancrage étant radialement déployable et le cadre de valvule étant radialement déployable et un diamètre de travail (58) le plus grand du cadre d'ancrage étant supérieur à un diamètre de travail le plus grand du cadre de valvule.

5. Système de valvule prothétique selon l'une quelconque des revendications 1 à 4, comprenant en outre une pluralité de cadres de valvule, chacun de la pluralité de cadres de valvule présentant un diamètre de travail le plus grand différent de chaque autre et étant conçu pour s'ancrer au cadre d'ancrage afin de supporter le cadre de valvule respectif à l'intérieur de la partie du corps du patient, une pluralité de feuillets valvulaires (68) étant accouplés au cadre de valvule respectif et conçus pour s'étendre radialement vers l'intérieur depuis une surface interne (44) du cadre de valvule respectif.

6. Système de valvule prothétique selon l'une quelconque des revendications 1 à 5, le cadre de valvule étant conçu pour être ancré au cadre d'ancrage à l'intérieur du corps du patient.

7. Système de valvule prothétique selon l'une quelconque des revendications 1 à 6, le cadre de valvule comprenant une pluralité de bras support (64) s'étendant axialement accouplés à la pluralité de feuillets valvulaires,
comprenant éventuellement en outre un ou plusieurs bras support (66) s'étendant de manière circonférentielle accouplant la pluralité de bras support s'étendant axialement les uns aux autres.

8. Système de valvule prothétique selon l'une quelconque des revendications 1 à 7, le cadre de valvule étant autodéployable et conçu pour se déployer radialement vers l'extérieur.

9. Système de valvule prothétique selon l'une quelconque revendication précédente, le deuxième élément de liaison (102) étant conçu pour se déplacer relativement axialement vers le premier élément de liaison (94) afin de venir en prise avec le premier élément de liaison en raison du déploiement radial du cadre de valvule.

10. Système de valvule prothétique selon l'une quelconque revendication précédente :
a.) le mécanisme de verrouillage étant conçu pour résister au déploiement axial de la structure d'ancrage ; et/ou
b.) le mécanisme de verrouillage étant conçu pour résister à une compression radiale du cadre de valvule et du cadre d'ancrage ; et/ou
c.) le mécanisme de verrouillage résistant au déploiement radial du cadre de valvule au-delà d'un diamètre (56).

11. Système de valvule prothétique selon l'une quelconque revendication précédente, le premier élément de liaison comprenant un bras (108) s'étendant axialement, une longueur (112) du bras définissant un diamètre au niveau duquel une compression radiale du cadre de valvule et du cadre d'ancrage est contrée par le mécanisme de verrouillage.

12. Système de valvule prothétique selon l'une quelconque revendication précédente, comprenant en outre une pluralité de cadres de valvule, chacun de la pluralité de cadres de valvule comprenant un élément de liaison présentant un bras (108) conçu pour se déplacer relativement axialement vers le deuxième élément de liaison (110) afin de venir en prise avec le deuxième élément de liaison, chaque bras respectif présentant une longueur (112) qui diffère de chaque autre et qui définit un diamètre au niveau duquel une compression radiale du cadre de valvule respectif et du cadre d'ancrage est contrée.

13. Système de valvule prothétique selon l'une quelconque revendication précédente, le premier élément de liaison ou le deuxième élément de liaison comprenant un ou plusieurs éléments parmi une broche (160), un verrou ou une ouverture (162).
